# EUROPÄISCHE PATENTSCHRIFT

(11) **EP 3 041 430 B1**
(45) Veröffentlichungstag und Bekanntmachung des Hinweises auf die Patenterteilung: **20.11.2019**
(21) Anmeldenummer: 14781054.3
(22) Anmeldetag: 04.09.2014
(51) Int. Cl.: A61B 90/00, A61B 17/34

(54) **VORRICHTUNG ZUR ANBRINGUNG VON MEDIZINISCHEN ZIELVORRICHTUNGEN U. DGL.**
DEVICE FOR ATTACHING MEDICAL TARGET DEVICES AND THE LIKE
DISPOSITIF DE FIXATION DE DISPOSITIFS MÉDICAUX DE CIBLAGE ET ANALOGUES

(30) Priorität: 04.09.2013 DE 202013007831 U
(43) Veröffentlichungstag der Anmeldung: 13.07.2016
(73) Patentinhaber: Isys Medizintechnik GmbH, 6370 Kitzbühel (AT)
(72) Erfinder: VOGELE, Michael, 86830 Schwabmünchen (DE)
(74) Vertreter: Fiener, Josef
(86) Internationale Anmeldenummer: PCT/EP2014/002399
(87) Internationale Veröffentlichungsnummer: WO 2015/032498

(56) Entgegenhaltungen:
- US-A- 3 457 922
- US-A1- 2002 133 174
- US-A1- 2003 125 753
- US-A1- 2007 276 407
- US-A1- 2009 069 945
- US-A1- 2010 137 680
- US-A1- 2012 184 956
- US-A1- 2012 266 898
- US-B2- 7 881 823

## Beschreibung

Die Erfindung betrifft eine Vorrichtung zur Anbringung von medizinischen Zielvorrichtungen u. dgl. nach den oberbegrifflichen Merkmalen des Anspruches 1.

Unter "Anbringung" sollen hierbei auch die Positionierung derartiger Instrumente verstanden werden, wobei insbesondere medizinische Zielvorrichtungen, Marker, Sonden und/oder chirurgische Instrumenten für bildgestützte, minimalinvasive Operationsverfahren zuverlässig eingestellt und fixiert werden sollen.

In vielen Bereichen der Humanmedizin oder der medizinischen Forschung ist eine sichere Fixierung oder (mechanische) Anbringung von Vorrichtungen oder Geräten erforderlich. Von größter Bedeutung ist dies insbesondere auf dem Gebiet der diagnostischen und therapeutischen Radiologie und/oder Onkologie, der Strahlentherapie oder bei operativen/chirurgischen Eingriffen (Neurochirurgie, HNO, Orthopädie, usw.).

Durch die Einbeziehung der Computertechnologie in Diagnose und Therapie sind die Anforderungen an Genauigkeit und Reproduzierbarkeit sowohl bei der Festlegung eines stereotaktischen Rahmensystems am Menschen als auch bei der Fixierung des Körpers selbst gestiegen. Komfort, Schnelligkeit in der Anwendung, Mobilität und Kosten spielen dabei eine erhebliche Rolle. Neben den herkömmlichen Fixationsarten am Körper mit Bändern oder Manschetten oder gar durch Verschraubung im Knochen sind auch Schalungen bekannt, die jedoch diverse Nachteile, insbesondere hinsichtlich der Fixationsgenauigkeit aufweisen. Andere Techniken wie Schienen, thermoplastisches Material, Kunststoffabdruck, Gipse, etc. weisen ähnliche Nachteile auf. Zusätzlich sind diese Methoden noch mit erheblichem finanziellen bzw. zeitlichen Aufwand verbunden und werden deshalb nur für Langzeitanwendungen verwendet. In der DE 20 2011 005 573 hat der Anmelder bereits ein Abformelement vorgeschlagen, das die vorstehenden Nachteile weitgehend vermeidet.

US 2009/069945 A1 offenbart eine Vorrichtung zur Anbringung von medizinischen Zielvorrichtungen, Markern, Sonden oder chirurgischen Instrumenten für bildgestützte, minimalinvasive Operationen.

Der Erfindung liegt deshalb die Aufgabe zugrunde, ergänzend hierzu eine Vorrichtung zur Anbringung von medizinischen Zielvorrichtungen zu schaffen, welche einfach im Aufbau und Anwendung und dabei in hohem Maße patientenschonend ist. Die Vorrichtung soll darüber hinaus die exakte Anbringung von Eichpunkten (sog. Markern) und eine optimale Zugänglichkeit zu Operationsgebieten ermöglichen.

Diese Aufgabe wird durch eine Vorrichtung mit den Merkmale des Anspruches 1 gelöst. Vorteilhafte Ausgestaltungen sind Gegenstand der Unteransprüche.

Die vorgeschlagene Vorrichtung zur Anbringung von medizinischen Zielvorrichtungen, Markern, Sonden oder chirurgischen Instrumenten für bildgestützte, minimalinvasive Operationen zeichnet sich dadurch aus, dass ein Trägerelement, das bevorzugt mit einer Klebeschicht auf der Körperoberfläche positionierbar ist, mindestens eine formschlüssige Halterung für eine Feinpositioniereinheit aufweist, die eine dazu passende Komplementärform besitzt. Hierdurch lässt sich in Art eines Schnellverschlusses die Feinpositioniereinheit rasch am Trägerelement fixieren, insbesondere einrasten oder festklemmen, so dass einer schneller und genauer Aufbau erzielt wird. Auch der Abbau nach der durchgeführten Operation kann rasch ausgeführt werden. Die formschlüssige Halterung ist hierbei bevorzugt auf der Hülle des Trägerelements aufgeklebt oder integriert (in dieses eingeklebt oder eingegossen). Diese Halterung weist insbesondere eine Hakenform mit dazu passender Nut oder komplementärer Kerbe und/oder einen Rastbolzen auf, so dass die Feinpositioniereinheit daran rasch eingeklickt oder eingerastet werden kann. Bevorzugt ist diese Komplementärform dabei an der Basisplatte der Feinpositioniereinheit, insbesondere deren Kreuzschlitten in robuster Weise integriert.

Das Trägerelement ist reversibel von einem formstabilen in einen formbaren Zustand überführbar ist, insbesondere durch Aufheben oder Anlegen eines Vakuums im Trägerelement. Die Feinpositioniereinheit besitzt zwei übereinander angeordnete Kreuzschlitten, mit denen die Feineinstellung der Instrumente, insbesondere Nadeln oder dgl. bei der Behandlung, insbesondere mittels Skalierungen erfolgen kann. Dies kann an der Feinpositioniereinheit mittels Stellrädern durchgeführt werden, die bevorzugt mit relativ großen Durchmessern ausgeführt sind, so dass diese seitlich über die Kreuzschlitten herausragen. Damit ist eine gut zugängliche, manuelle Bedienung möglich. Dabei kann zur leichteren Feinpositionierung auch ein optischer Sensor in Form eines Laserpointer zur Darstellung des Zielpunkts auf der Patientenhaut verwendet werden.

Die Feinpositioniereinheit bzw. deren Kreuzschlitten können auch über Bowdenzüge oder Stellspindeln mit biegsamen Wellen aus einigen Metern bedienbar sein oder auch hydraulisch/pneumatisch fernbedienbar sein. Hierdurch wird die Strahlenbelastung des Operateurs erheblich reduziert. Die Halterung und/oder das Trägerelement kann auch über einen Verstellarm zum Behandlungstisch hin fixiert werden, wobei die Halterung bei der Anwendung im Rückenbereich bevorzugt plattenförmig ausgebildet ist, insbesondere in T-Form. Die Halterungsplatte ist insbesondere röntgentransparent und besteht bevorzugt aus Carbon, um durch die hohe Steifigkeit dieses Materials die Stabilität der Vorrichtung zu erhöhen. Um eine hygienische Handhabung zu gewährleisten, sind bevorzugt mehrere Komponenten der Vorrichtung als sterile Einwegartikel ausgebildet. Zudem können an der Halterung und/oder Feinpositioniereinheit auch Kraftsensoren vorgesehen sein, um Atem- oder Organbewegungen zu erfassen und z.B. den Vorschub einer Punktionsnadel nur in bestimmten Bewegungsphasen zuzulassen.

Ausführungsbeispiele werden anhand der Zeichnungen nachfolgend beschrieben. Hierbei zeigen:
- Fig. 1: eine Gesamtansicht der Vorrichtung an einem Patienten;
- Fig. 2: eine vergrößerte Perspektivansicht der Feinpositioniereinheit;
- Fig. 3: eine Draufsicht auf die Feinpositioniereinheit;
- Fig. 4: eine über der Halterung dargestellte Feinpositioniereinheit;
- Fig. 5: eine Feinpositioniereinheit mit Bowdenzug; und
- Fig. 6: eine Feinpositioniereinheit mit X-/Y-Stellspindeln.

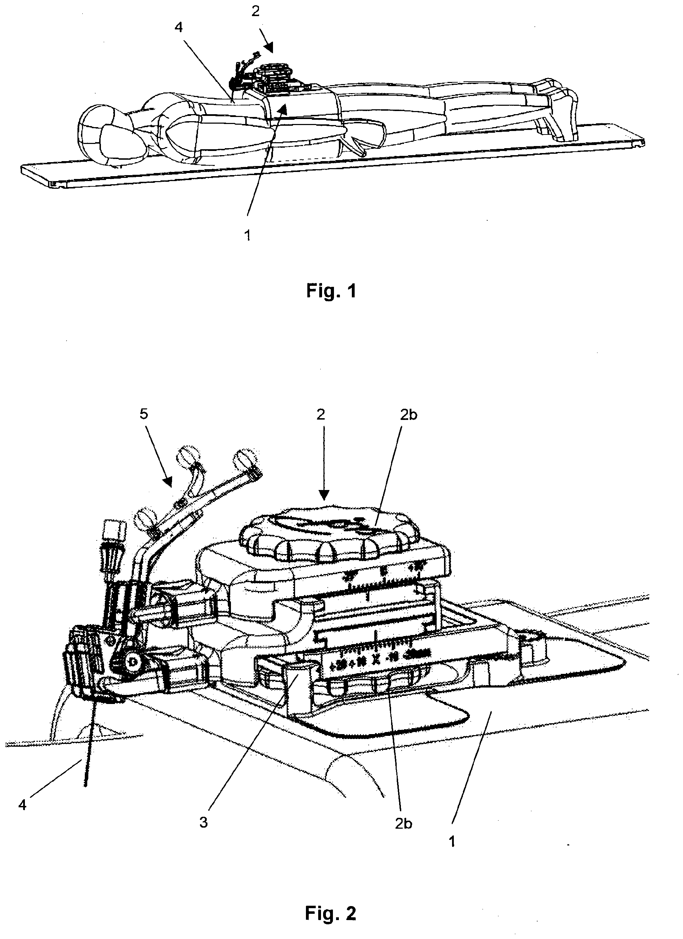

In Fig. 1 ist die vorgeschlagene Vorrichtung an einem Patienten auf einer Behandlungsliege dargestellt. Ein Trägerelement 1 umgreift dabei den Rückenbereich des Patienten tunnelartig. Dies erfolgt bevorzugt mit einer Adhäsivschicht, die mit dem Trägerelement 1 verbunden ist und an die Haut angedrückt wird. Die Adhäsivschicht kann auch durch einen Sprühkleber gebildet sein, der auf die Haut und/oder die körperseitige Fläche des Trägerelements 1 aufgesprüht wird. Dann wird bevorzugt eine Vakuumpumpe angeschlossen und das kissenartige Trägerelement 1 evakuiert. Hierdurch wird eine formstabile Schale gebildet, auf der über eine (weiter unten beschriebene) formschlüssige Halterung 3 (vgl. insbes. Fig, 4) eine Feinpositioniereinheit 2, hier zur Justierung einer Nadel 4 aufgesetzt ist. In dieser Position liegt das Trägerelement 1 mit hoher Haltekraft am Rücken (oder im Schulterbereich) an. Erst nach Aufheben des Vakuums lässt sich dieses wieder abziehen. Somit lässt sich das Trägerelement 1 vom formstabilen Fixierungszustand wieder in einen weichen, formbaren Zustand zurückführen. Hierbei können auch mehrere, voneinander unabhängige Vakuumkammern vorgesehen sein, um die Vorrichtung z. B. partiell zu destabilisieren bzw. zu stabilisieren. Solche Vakuumkammern sind z. B. aus der US 7,881,823 B2 bekannt, deren Gesamtaufbau jedoch sehr sperrig ist, während sich die vorgeschlagene Vorrichtung durch ihre kompakte Bauweise auszeichnet.

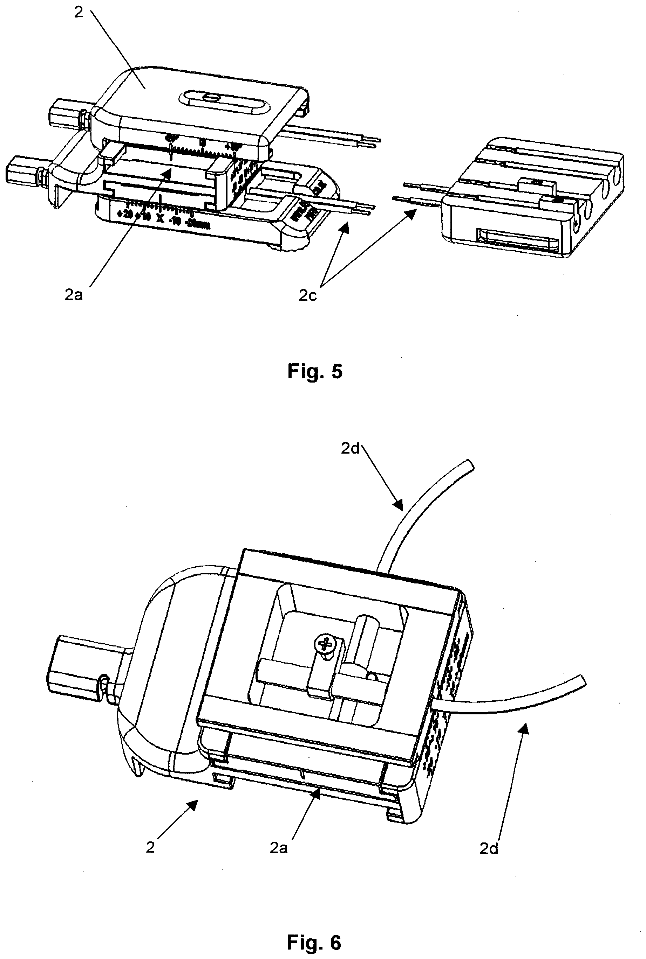

Die Hülle des Trägerelements 1 ist bevorzugt gewebe- oder folienartig ausgebildet und mit Granulat, insbesondere Kunststoffkügelchen gefüllt ist. Wie in Fig. 2 dargestellt, ist an der Außenseite des verfestigten Trägerelements 1 eine plattenförmige Halterung 3, bevorzugt aus Carbon (CfK) angebracht, insbesondere verklebt oder mechanisch fixiert. Zur weiteren Fixierung an einem Operationstisch kann auch ein Schwenkarm vorgesehen sein. In Fig. 2 ist an der Feinpositioniereinheit 2 weiterhin ein dreiarmiger Marker 5 oberhalb der Punktionsnadel 4 dargestellt. Zudem ist der Aufbau mit zwei Kreuzschlitten 2a (vgl. auch Fig. 5) und die Anbringung von Skalierungen zur manuellen Einstellung ersichtlich. Diese Feinjustage erfolgt hier mit zwei großen Stellrädern 2b.

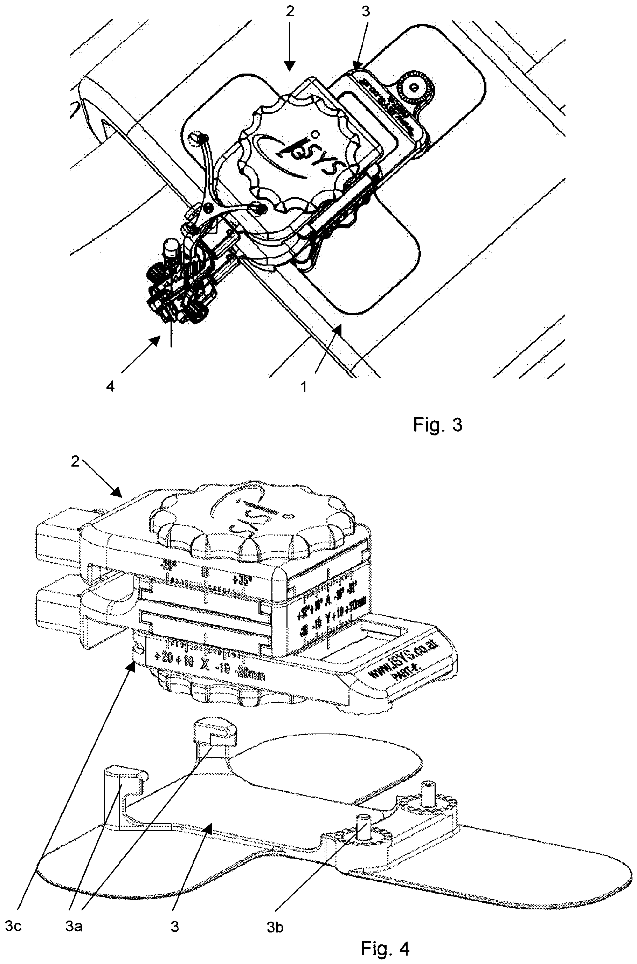

Wie in Fig. 3 dargestellt, weist die Halterung 3 eine T-Grundform auf, auf der die Feinpositioniereinheit 2 formschlüssig befestigt oder verrastet ist. Dies erfolgt bevorzugt mit Haken 3a, die insbesondere in Fig. 4, links ersichtlich sind. Diese Haken oder Vorsprünge greifen dabei in entsprechende Schlitze oder komplementäre Kerben 3c an der Feinpositioniereinheit 2, so dass diese zum Trägerelement 1 hin sicher fixiert ist. Hierzu dienen auch Rastbolzen 3b, mit denen die Feinpositioniereinheit 2 sicher und schnell verankert werden kann. Das Abnehmen nach erfolgter Behandlung (z. B. Rückmarkspunktion im Lendenwirbelbereich mit der Nadel 4 unter Navigation mit dem Marker 5) kann ebenso rasch erfolgen, insbesondere auch während der Behandlung bei plötzlichen Panikattacken des Patienten .

In Fig. 5 und 6 sind Alternativen zur Verstellung der Feinpositioniereinheit 2 gezeigt, nämlich in Fig. 5 mit Bowdenzügen 2c und in Fig. 6 mittels Stellspindeln, die über flexible Wellen 2d fernbedienbar sind. Dabei kann der behandelnde Arzt (oder andere Fachpersonen) die X-Y-Verstellung der Feinpositioniereinheit 2 aus sicherer Entfernung von mehreren Meter vornehmen. Hierzu können neben den Skalierungen am Kreuzschlitten 2a auch Displays oder Bildschirme vorgesehen sein. Ebenso sind hydraulische oder pneumatische Betätigungen denkbar, wobei die Bauteile bevorzugt metallfrei hergestellt sind. Somit kann die Vorrichtung gut in der Röntgenstrahlung oder auch bei der MR-Bildgebung eingesetzt werden. Der Bedienblock für die Bowdenzüge 2c (in Fig. 5, rechts) braucht hierbei nicht röntgentransparent sein oder MR- kompatibl sein, da sich diese Bauteile außerhalb des Strahlungsfeldes befinden. Bei hydraulischer Betätigung (über Schläuche analog zu den in Fig. 6 gezeigten, flexiblen Wellen 2d) können die Schläuche bzw. Zylinder auch mit Kontrastmittel gefüllt sein. Durch 2D/3D-Bildaufnahmen kann so das Volumen in den Zylindern überwacht werden. Aus dem Volumen in den Zylindern kann wiederum die exakte Position der X-/Y-Schlitten bestimmt werden. Neben der Positionsberechnung kann die Flüssigkeit in den Zylindern auch zur Registrierung der Nadelposition verwendet werden. Dabei dient der Zylinder quasi als Marker, mit deren Hilfe man Zielkoordinaten im 2D/3D-Bilddatensatz berechnen kann. Mit Echtzeitbildgebung ist es auch möglich, über eine Echtzeit-Berechnung der Zylindervolumina einen geschlossenen Regelkreis zu errichten.

Dabei werden Ist- und Sollposition (Zielpunkt bzw. Zylindervolumen) solange nachgeregelt bis die Werte in dem vorgegebenen Toleranzbereich liegen. Zudem können an der Feinpositioniereinheit 2 auch Kraftsensoren oder optische Sensoren vorgesehen sein, um die Behandlung zu steuern bzw. zu überwachen.

Insgesamt können die vorstehend beschriebenen Antriebskonzepte auch miteinander kombiniert werden. So sind auch Mischformen aus manuellen und motorischen Einheiten und Untereinheiten denkbar (z.B. zum halbautomatisches Ansteuern einer Zielposition). Es ist auch denkbar, dass eine oder mehrere Schlittenebenen frei pendeln, um z.B. Kollisionen mit Patient/Umgebung bei aktiver Bewegung in Art einer Überlastsicherung einer oder mehrerer Achsen auszugleichen. An der Feinpositioniereinheit 2 können auch mehrere Instrumente vorgesehen sein, ebenso auch Marker-/Referenzvorrichtungen zur (automatischen) Erkennung durch bildgebende Verfahren sowie optische oder magnetische Messsysteme.

## Patentansprüche

1. Vorrichtung zur Anbringung von medizinischen Zielvorrichtungen, Markern, Sonden oder chirurgischen Instrumenten für bildgestützte, minimalinvasive Operationen, mit mindestens einem Trägerelement (1), das auf der Hautoberfläche eines Patienten positionierbar ist, und einer Feinpositioniereinheit (2), die eine formschlüssige Halterung (3) aufweist und eine dazu passende Komplementärform (3c) besitzt,
**dadurch gekennzeichnet, dass**
das Trägerelement (1) kissenartig geformt und von einem formstabilen in einen formbaren Zustand und umgekehrt überführbar ist, insbesondere durch Aufheben oder Anlegen eines Vakuums im Trägerelement (1), sowie die Feinpositioniereinheit (2) wenigstens zwei übereinander angeordnete Kreuzschlitten (2a) aufweist.

2. Vorrichtung nach Anspruch 1, **dadurch gekennzeichnet, dass** die Anbringung des Trägerelements (1) am Patienten durch Vakuum und/oder Adhäsion erfolgt.

3. Vorrichtung nach Anspruch 1 oder 2, **dadurch gekennzeichnet, dass** die formschlüssige Halterung (3) auf der Hülle des Trägerelements (1) aufgeklebt oder integriert ist,

4. Vorrichtung nach einem der Ansprüche 1 bis 3, **dadurch gekennzeichnet, dass** die Halterung (3) wenigstens eine Hakenform (3a) und/oder einen Rastbolzen (3b) aufweist.

5. Vorrichtung nach einem der Ansprüche 1 bis 4, **dadurch gekennzeichnet, dass** die Feinpositioniereinheit (2) einer Überlastsicherung aufweist.

6. Vorrichtung nach einem der Ansprüche 1 bis 5, **dadurch gekennzeichnet, dass** die Feinpositioniereinheit (2) durch Stellräder (2b) verstellbar ist,

7. Vorrichtung nach einem der Ansprüche 1 bis 5, **dadurch gekennzeichnet, dass** die Feinpositioniereinheit (2) über Bowdenzüge (2c) oder Stellspindeln (2d) mit biegsamen Wellen und/oder hydraulisch/pneumatisch/motorisch fernbedienbar ist, insbesondere in mehreren Achsen.

8. Vorrichtung nach einem der Ansprüche 1 bis 7, **dadurch gekennzeichnet, dass** die Halterung (3) plattenförmig ausgebildet ist, insbesondere in T-Form.

9. Vorrichtung nach einem der Ansprüche 1 bis 8, **dadurch gekennzeichnet, dass** zumindest Komponenten der Vorrichtung als sterile Einwegartikel ausgebildet sind.

10. Vorrichtung nach einem der Ansprüche 1 bis 9, **dadurch gekennzeichnet, dass** die Feinpositioniereinheit (2) und/oder Antriebsmodule davon Skalierungen aufweist.

11. Vorrichtung nach einem der Ansprüche 1 bis 10, **dadurch gekennzeichnet, dass** die Feinpositioniereinheit (2) Kraftsensoren und/oder optische Sensoren aufweist.

## Claims

1. Device for attachment of medical target devices, markers, probes or surgical instruments for image-assisted, minimally invasive surgery, with at least one carrier element (1) which can be positioned on the body surface of a patient, and a fine-positioning unit (2) having an interlocking mount (3) and a matching complementary shape (3c),
**characterized in that**
the carrier element (1) is formed in a cushion shape and can be moved from a dimensionally stable into a formable state, and vice versa, in particular by releasing or applying vacuum in the carrier element (1), and wherein the fine-positioning unit (2) has at least two superposed cross slides (2a).

2. Device according to claim 1, **characterized In that** the carrier element (1) is attached to the patient by vacuum and/or adhesion.

3. Device according to claim 1 or 2, **characterized In that** the interlocking mount (3) is glued on the envelope of the carrier element (1) or is integrated,

4. Device according to one of claims 1 to 3, **characterized in that** the mount (3) includes at least one hook-shape (3a) and/or a latching pin (3b).

5. Device according to one of claims 1 to 4, **characterized in that** the fine-positioning unit (2) comprises an overload safety device.

6. Device according to one of claims 1 to 5, **characterized in that** the fine-positioning unit (2) is adjustable by means of adjusting wheels (2b).

7. Device according to one of claims 1 to 5, **characterized in that** the fine-positioning unit (2) is remotely controlled via Bowden cables (2c) or adjusting spindles (2d) with flexible shafts and/or a hydraulic/pneumatic motor, in particular in multiple axes.

8. Device according to one of claims 1 to 7, **characterized in that** the mount (3) is plate-shaped, in particular in T-shape.

9. Device according to one of claims 1 to 8, **characterized in that** at least components of the device are formed as a sterile disposable article.

10. Device according to one of claims 1 to 9, **characterized in that** the fine-positioning unit (2) and/or drive modules thereof comprise scalings.

11. Device according to one of claims 1 to 10, **characterized in that** the fine-positioning unit (2) comprises force sensors and/or optical sensors.

## Revendications

1. Dispositif dévolu à la mise en place d'appareils médicaux de ciblage, de marqueurs, de sondes ou d'instruments chirurgicaux conçus pour des opérations mini-invasives à assistance par images, comprenant au moins un élément de support (1) pouvant être positionné sur la surface de la peau d'un patient, et une unité (2) de positionnement précis qui est pourvue d'un élément de retenue (3) à engagement positif et possède une forme complémentaire (3c) adaptée à ce dernier,
**caractérisé par le fait que**
l'élément de support (1) présente une configuration de type coussin et peut être amené d'un état de forme stable à un état déformable, et inversement, en particulier par suppression ou création d'une dépression dans ledit élément de support (1), l'unité (2) de positionnement précis étant dotée d'au moins deux chariots croisés (2a) placés en superposition.

2. Dispositif selon la revendication 1, **caractérisé par le fait que** la mise en place de l'élément de support (1), sur le patient, a lieu par dépression et/ou par adhérence.

3. Dispositif selon la revendication 1 ou 2, **caractérisé par le fait que** l'élément de retenue (3) à engagement positif est collé sur l'enveloppe de l'élément de support (1), ou intégré.

4. Dispositif selon l'une des revendications 1 à 3, **caractérisé par le fait que** l'élément de retenue (3) est au moins muni d'une configuration crochue (3a) et/ou d'un tenon d'encliquetage (3b),

5. Dispositif selon l'une des revendications 1 à 4, **caractérisé par le fait que** l'unité (2) de positionnement précis est équipée d'une sûreté prévenant les surcharges.

6. Dispositif selon l'une des revendications 1 à 5, **caractérisé par le fait que** l'unité (2) de positionnement précis peut être réglée au moyen de roues de réglage (2b).

7. Dispositif selon l'une des revendications 1 à 5, **caractérisé par le fait que** l'unité (2) de positionnement précis peut être manœuvrée à distance par l'intermédiaire de câbles Bowden (2c) ou de broches de réglage (2d) à arbres souples, et/ou en mode hydraulique/pneumatique/motorisé, en particulier suivant plusieurs axes.

8. Dispositif selon l'une des revendications 1 à 7, **caractérisé par le fait que** l'élément de retenue (3) est réalisé en forme de plaque notamment configurée en T.

9. Dispositif selon l'une des revendications 1 à 8, **caractérisé par le fait qu'**au moins des composants dudit dispositif sont réalisés sous la forme d'articles stériles à usage unique.

10. Dispositif selon l'une des revendications 1 à 9, **caractérisé par le fait que** l'unité (2) de positionnement précis, et/ou des modules d'entraînement de cette dernière, présentent) des graduations,

11. Dispositif selon l'une des revendications 1 à 10, **caractérisé par le fait que** l'unité (2) de positionnement précis est équipée de capteurs de forces et/ou de capteurs optiques.
